(19) Europäisches Patentamt
European Patent Office
Office européen des brevets

(11) Numéro de publication : **0 399 909 B1**

# (12) FASCICULE DE BREVET EUROPEEN

(45) Date de publication du fascicule du brevet : **04.08.93 Bulletin 93/31**

(51) Int. Cl.[5] : **A61K 7/48,** A61K 31/70, A61K 35/28

(21) Numéro de dépôt : **90401380.2**

(22) Date de dépôt : **23.05.90**

## (54) Composition cosmétique s'opposant au vieillissement de la peau.

(30) Priorité : **23.05.89 FR 8906747**

(43) Date de publication de la demande : **28.11.90 Bulletin 90/48**

(45) Mention de la délivrance du brevet : **04.08.93 Bulletin 93/31**

(84) Etats contractants désignés : **AT BE CH DE DK ES FR GB GR IT LI LU NL SE**

(56) Documents cités :
**EP-A- 0 318 393**
**DE-A- 2 330 902**
**FR-A- 2 574 661**

(73) Titulaire : **ELF SANOFI**
**32-34, rue Marbeuf**
**F-75008 Paris (FR)**

(72) Inventeur : **Le Fur, Gérard**
**19 ter, Rue des Carrières**
**F-95160 Montmorency (FR)**
Inventeur : **Sabadie, Michel**
**Rue de la Fontaine St. Germain**
**F-27500 Bernay (FR)**

(74) Mandataire : **Gillard, Marie-Louise et al**
**Cabinet Beau de Loménie 158, rue de l'Université**
**F-75340 Paris Cédex 07 (FR)**

EP 0 399 909 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne une composition cosmétique s'opposant au vieillissement de la peau, à l'aide d'un système générateur d'adémétionine.

Adémétionine est la Dénomination Commune Internationale du sel interne de l'hydroxyde de (S)-5'-[(3-amino-3-carboxypropyl)-méthylsulfonio]-5'-désoxyadénosine de formule

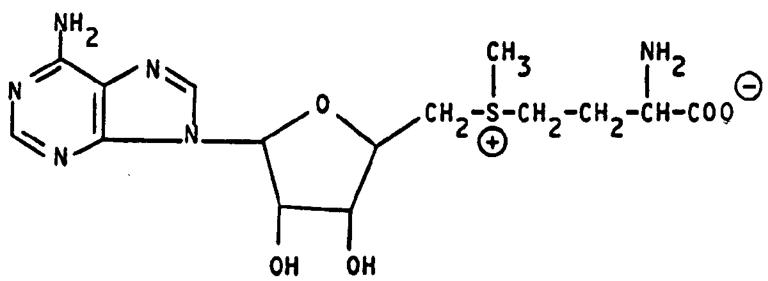

(I)

appelé également S-adénosyl-L-méthionine ou plus simplement SAMe.

L'adémétionine est une molécule physiologique de distribution quasi ubiquitaire dans les tissus et dans les liquides de l'organisme où elle intervient dans des processus biologiques importants comme donneur de groupes méthyle dans de nombreuses réactions de transméthylation et comme précurseur de composés soufrés physiologiques tels que le glutathion, la cystéine, la taurine, le CoA.

Il est connu que les taux d'adémétionine sont élevés chez l'enfant et l'adolescent alors qu'ils diminuent chez l'adulte et se réduisent ultérieurement à l'âge présénile et sénile.

On a trouvé (FR-A-2623396 publié le 26 mai 1989) que l'adémétionine possède une action s'opposant au vieillissement de la peau et qu'elle peut être utilisée dans des buts cosmétiques directement sur la peau.

L'action s'opposant au vieillissement est due aux propriétés biochimiques de l'adémétionine. La transméthylation se vérifie sur des molécules biologiques, notamment les protéines de la peau, les acides nucléiques et les phospholipides, qui sont biotransformés et entrent dans des cycles anaboliques et cataboliques. Cette activité ana-catabolisante au niveau des cellules épidermiques favorise leur régénération, alors que l'action sur les méthyltransférases, par l'augmentation de la méthylation des phospholipides, rend plus fluides les membranes cellulaires, ralentissant ainsi le processus naturel de vieillissement de la peau.

Cette action fluidifiant les membranes a été mise en évidence sur des cellules de l'épiderme humain. La microviscosité de la membrane des cellules épidermiques humaines a été étudiée selon la méthode de Shinitzky et al. (J. Biol. Chem. 1974, 249, 2652-2657) en utilisant le diphénylhexatriène comme marqueur fluorescent. La présence d'adémétionine à une concentration molaire de $10^{-6}$ à $10^{-4}$ diminue la viscosité membranaire de 10 à 25 %.

Toutefois, dans le but d'une utilisation cosmétique, l'adémétionine présente l'inconvénient d'être instable et peu apte à la manipulation dans l'industrie cosmétique.

On a maintenant trouvé, dans des essais in vitro, que la bétaïne, même à des concentrations très basses, agit significativement sur la quantité d'adémétionine des fibroblastes de peau humaine.

On a aussi trouvé qu'il est possible d'obtenir les mêmes effets que ceux observés avec l'adémétionine en traitant la peau avec de la bétaïne, de l'ATP ou un système générateur d'ATP, un sel de potassium et un sel de magnésium, dans un excipient cosmétique, en générant ainsi l'adémétionine in situ.

Ainsi, la présente invention concerne une composition cosmétique caractérisée en ce qu'elle renferme un mélange de

(a) bétaïne ;
(b) ATP ou un système générateur d'ATP (SG-ATP) ;
(c) un sel de magnésium ; et
(d) un sel de potassium

dans un excipient cosmétique.

Par bétaïne", on entend le composé de formule $(CH_3)_3N^+\text{-}CH_2\text{-}COO^-$, ainsi que ses sels d'addition avec des acides dermocompatibles et les hydrates correspondants.

Par "système générateur d'ATP" ou "SG-ATP", on entend tout extrait biologique capable d'augmenter la

respiration cellulaire au sein de la mitochondrie, en accélérant ainsi le métabolisme de la cellule et, par conséquent, la transformation des produits issus de la glycolyse et du cycle de Krebs en ATP. En augmentant la respiration cellulaire, dans le stade ultime de la chaîne respiratoire, on génère de l'ATP, d'abord directement par transformation biochimique du glucose en ATP, et puis par l'intermédiaire de l'acide pyruvique qui, à son tour, génère de l'ATP (LEHNINGER - Principes biochimiques, ed. Flammarion Medecine Science, 1985, 497-498).

Parmi ces extraits, les extraits de cellules eucaryotes, en particulier de levures, obtenus par des procédés d'extraction et de purification conventionnels qui permettent de garder presque intacts les composants biochimiques, notamment les aminoacides, les peptides et les enzymes, présents dans les cellules, sont particulièrement préférés. Ces extraits sont connus en littérature et ils sont aussi disponibles dans le commerce, par exemple sous la dénomination VITACELL LS 1917 (produit par la société française Laboratoires Sérobiologiques S.A.). Parmi les extraits qui peuvent être employés, on préfère également, selon l'invention, ceux de la rate bovine, bien connus en littérature et commercialisés, par exemple sous les marques REVITALINE (produit par la société suisse Pentapharm) et OXYDERMINE (produit par la société française Sederma). Ils sont obtenus par des procédés d'extraction et de purification, connus en tant que tels, qui permettent également de garder presque intacts les composants biochimiques présents dans les cellules.

Comme sels de magnésium et de potassium, tout sel organique ou inorganique peut être utilisé, à condition qu'il soit dermocompatible. Les gluconates, les pyroglutamates et les chlorures sont les anions préférentiels.

La demande de brevet anglais GB-A-2151924 décrit une composition cosmétique ou dermatologique pour le soin de la peau qui contient comme composants essentiels un extrait végétal de Oenothera biennis et un extrait de la rate. Tandis que l'huile d'Oenothera est utilisée pour former une barrière sur l'épiderme, régulant ainsi la perte d'eau de la peau, l'extrait de rate est employé afin d'activer la respiration cellulaire, effet par ailleurs bien connu en littérature et décrit ci-dessus.

La demande de brevet français FR-A-2609393 revendique des compositions pharmaceutiques ou cosmétiques, ou des compositions utiles comme matière de base pour la préparation de compositions pharmaceutiques ou cosmétiques contenant une substance azotée telle qu'un aminoacide, un oligo- ou poly-peptide, une protéine ou un dérivé de ceux-ci, y compris la bétaïne. D'après cette demande de brevet français, qui de toute façon ne décrit pas une composition telle que celle de la présente invention, ces compositions présentent, selon la forme sous laquelle elles sont formulées, une activité à la fois hydratante, nourrissante, régénératrice, protective, stimulante de la croissance des cellules épidermiques, dermiques, et du bulbe pileux.

Dans la composition cosmétique de la présente invention, les composants peuvent être présents dans les proportions suivantes (les pourcentages exprimés s'entendent en poids) :

(a) bétaïne de 0,001 à 1 %
(b) ATP (ou SG-ATP) de 0,045 à 4,5 %
(c) $Mg^{++}$ de 10 ppb à 0,3 %
(d) $K^{+}$ de 0,0001 à 0,6 %

On utilise, de préférence, les composants dans les pourcentages pondéraux suivants :

(a) bétaïne de 0,03 à 0,3 %
(b) ATP (ou SG-ATP) de 0,15 à 1,5 %
(c) $Mg^{++}$ de 15 ppb à 0,1 %
(d) $K^{+}$ de 0,001 à 0,2 %

Les compositions les plus intéressantes sont celles qui contiennent les constituants suivants dans les pourcentages pondéraux ci-après :

(a) bétaïne de 0,05 à 0,25 %
(b) ATP (notamment les extraits de levure tels que par exemple le VITACELL LS 1917, ou les extraits de la rate bovine tels que par exemple la REVITALINE et la OXYDERMINE) de 0,25 à 1 %
(c) $Mg^{++}$ de 20 ppb à 0,06 %
(d) $K^{+}$ de 0,002 à 0,1 %

Pour la préparation des compositions cosmétiques selon la présente invention, les composants sont mélangés avec des excipients cosmétiques pour préparer des crèmes, des lotions, des émulsions ou des solutions. La préparation desdites compositions constitue un autre objet de l'invention.

En particulier, les composants sont mélangés aux excipients généralement employés dans la technique cosmétique tels que, par exemple, les matières grasses d'origine animale ou végétale, les huiles végétales, les acides gras, les alcools, les glycols polyalkyléniques, les cires, les hydrocarbures, les polyesters et, lorsqu'ils sont compatibles, peuvent être associés à l'eau et à des agents gélifiants. Dans ces préparations, on peut ajouter d'autres ingrédients compatibles avec les composants comme des agents conservateurs tels que les esters de l'acide 4-hydroxybenzoïque, des antioxydants, comme le butylhydroxytoluène ou les dérivés de la vitamine E, ou des parfums.

Les acides gras utilisés comme adjuvants dans les compositions cosmétiques de la présente invention peuvent être saturés ou insaturés contenant de 10 à 22 atomes de carbone, non substitués ou substitués avec un groupe hydroxyle, sous forme libre ou d'un de leurs sels alcalins.

La forme des compositions cosmétiques selon l'invention peut notamment être une crème dans laquelle les composants sont associés aux excipients couramment utilisés dans la cosmétologie, et compatibles avec lesdits composants, tels que la lanoline, ou ses dérivés.

Les compositions cosmétiques de l'invention peuvent être également présentées sous forme de gel dans les excipients appropriés tels que les esters de cellulose, les polymères acryliques, les esters d'acides gras, par exemple le palmitate ou le stéarate d'octyle ou d'autres agents gélifiants.

Selon un autre aspect, les principes actifs cités précédemment sont - au moins en partie - encapsulés dans des vecteurs phospholipidiques, sous forme de phases lamellaires, de vésicules sphériques ou de tubulures allongés, constitués par des couches de phospholipides alternées à des couches aqueuses, qui sont désignés par le terme général de "liposomes". Le choix des différents phospholipides à utiliser dans la préparation des liposomes, ainsi que les méthodes pour leur préparation et leur utilisation dans la préparation des compositions liposomées selon la présente invention, sont aisément réalisés en suivant les renseignements mentionnés en littérature (voir par ex. "Liposomes" - Ed. M. Ostro, Marcel Decher, New York, 1983).

Les compositions cosmétiques suivant l'invention peuvent aussi prendre la forme de lotion, de solution ou de microémulsion dans lesquelles les composants sont dissous ou microdispersés.

La forme des compositions cosmétiques suivant l'invention peut donc être une microdispersion de composants dans un liquide contenant de l'eau, de l'huile ainsi qu'un ou plusieurs agents tensioactifs. Ces dispersions présentent les caractères des microémulsions et ont pratiquement l'aspect de solutions vraies. Elles peuvent être préparées extemporanément.

Ces microémulsions jouissent d'une bonne stabilité et peuvent être conservées pendant le temps nécessaire pour l'utilisation à des température comprises entre 0 et 60°C sans qu'il y ait sédimentation des constituants ou séparation de phases de façon irréversible.

Les tensioactifs de la compositions sont choisis parmi les agents de surface utilisables en cosmétologie. On peut indiquer à titre d'exemples non limitatifs : les esters de sorbitol et leurs dérivés polyoxyéthylénés, les huiles de ricin (hydrogénées ou non) polyoxyéthylénées, les copolymères séquencés oxyde d'éthylène/oxyde de propylène, les alcools gras et les stérols polyoxyéthylénés, le laurylsulfate de sodium, le dioctylsulfosuccinate de sodium, les lécithines d'oeuf ou de soja, les huiles de silicones polyoxyéthylénées.

L'effet exercé par la bétaïne sur la formation de SAMe a été mis en évidence par les résultats obtenus à des concentrations croissantes de bétaïne dans des fibroblastes de peau humaine (ATCC No CRL 1513) en culture. Plus précisément, les cellules ($2.10^4$/ml) ont été mises en culture pendant 6 jours dans 250 ml de milieu de culture Dulbecco's Modified Eagle Medium (DMEM) contenant 20 % de sérum de veau foetal, en présence de concentrations croissantes (de $10^{-5}$ M à $5.10^{-4}$ M) de bétaïne. Au terme de la culture, les cellules ont été décollées à la trypsine (0,05 %) et à l'EDTA (0,02 %) et centrifugées. Le surnageant a été récupéré et précipité à l'acide trichloroacétique (0,2 N, v/v) puis centrifugé à nouveau. Le surnageant a été ensuite récupéré et lyophylisé. Les lyophylisats ont été repris avec de l'eau et analysés en HPLC (Colonne : Nucléosyl C8 ; Eluant : Solvant A (acétate de sodium 8,2 g ; octylsulfonate de sodium 200 mg ; acide citrique 4,2 g ; EDTA 50 mg ; eau q.s. pour 1 litre) 95 % et Solvant B (méthanol) 5 %) en utilisant un détecteur à 260 nm. La quantité de SAMe a été déterminée d'après une gamme étalon traitée dans les mêmes conditions expérimentales.

| | | | | | |
|---|---|---|---|---|---|
| Concentration de bétaïne ($10^{-5}$ M) | 0 | 1 | 5 | 10 | 50 |
| Concentration de SAMe (nMoles/$10^7$ cellules) | 0,54 | 0,62 | 0,94 | 1,72 | 1,90 |

En utilisant les compositions cosmétiques de la présente invention contenant, outre la bétaïne, l'ATP ou un générateur d'ATP, et des sels de magnésium et de potassium, on remarque après trente jours d'application régulière une nette amélioration de l'aspect de la peau et un ralentissement de la formation des rides.

Les compositions cosmétiques de la présente invention sont donc essentiellement destinées à :

- ralentir le vieillissement en maintenant une fluidité optimale des membranes des cellules cutanées, une fluidité membranaire élevée favorisant des échanges intercellulaires internes, donc un métabolisme optimum ;
- améliorer l'état des peaux vieillies prématurément par l'action de facteurs exogènes, selon le processus

ci-dessus.

En outre, les compositions cosmétiques de la présente invention sont très bien tolérées ; elles ne présentent aucune phototoxicité et leur application sur la peau pour des périodes de temps prolongées n'implique aucun effet systémique. Les exemples suivants illustrent l'invention sans toutefois la limiter.

Dans un souci de simplification, certains constituants des compositions ont été désignés par leur dénomination commerciale ou par un sigle dont voici les significations :

| | |
|---|---|
| VITACELL LS 1917 | : extrait de levures commercialisé par les Laboratoires Sérobiologiques S.A. |
| REVITALINE | : extrait de rate bovine commercialisé par PENTAPHARM |
| OXYDERMINE | : extrait de rate bovine commercialisé par SEDERMA |
| Solutol HS 15 | : 12-hydroxystéarate de polyéthylèneglycol 600, commercialisé par BASF |
| Cetiol HE | : polyéthylèneglycol-7 glycéryl cocoate, commercialisé par HENKEL |
| Labrafac hydrophile | : triglycérides contenant 7-8 atomes de carbone polyoxyéthylénés, commercialisé par GATTEFOSSE |
| Abil 8851 B | : diméthicone copolyol, commercialisé par GOLDSCHMIDT |
| Carbopol 934<br>Carbopol 940 | } carboxypolyméthylènes, commercialisés par GOLDSCHMIDT |
| Tween 60 | : Monostéarate de sorbitan éthoxylé |
| Tween 20 | : Laurate de sorbitan éthoxylé |
| EDTA | : acide éthylènediaminotétracétique |
| Filtre UVB | : 4-méthoxycinnamate d'éthyle-2 hexyle (marque PARSOL MCX). |

EXEMPLE 1

PREPARATION EXTEMPORANEE

A) Solvant :

| | | |
|---|---|---|
| carboxyméthylcellulose | | 0,30 g |
| conservateur dans propylèneglycol : | | 5,00 g |
| phénoxyéthanol | 0,5 g | |
| 4-hydroxybenzoate de méthyle | 0,1 g | |
| 4-hydroxybenzoate d'éthyle | 0,1 g | |
| 4-hydroxybenzoate de propyle | 0,1 g | |
| 4-hydroxybenzoate de butyle | 0,1 g | |
| propylèneglycol | 4,1 g | |
| huile de ricin hydrogénée éthoxylée | | 1,00 g |
| parfum | | 0,20 g |
| eau déminéralisée q.s.p. | | 100,00 g |

B) Poudre :

| | |
|---|---|
| bétaïne | 0,10 g |
| chlorure de magnésium | 0,05 g |
| chlorure de potassium | 0,10 g |
| REVITALINE | 0,50 g |
| lactose q.s.p. | 100,00 g |

EXEMPLE 2

CREME DE JOUR PROTECTRICE

| | |
|---|---|
| bétaïne | 0,05 g |
| chlorure de magnésium | 0,05 g |
| chlorure de potassium | 0,10 g |
| REVITALINE | 0,50 g |
| Tween 60 | 2,60 g |
| huile de silicone | 1,00 g |
| alcool cétylique | 2,00 g |
| huile minérale | 3,00 g |
| alcool de lanoline | 1,00 g |
| perhydrosqualène | 1,00 g |
| monostéarate de sorbitan | 2,40 g |
| palmitate de cétyle | 3,00 g |
| palmitate d'isopropyle | 4,00 g |

| | | |
|---|---|---|
| filtre UVB | | 2,00 g |
| EDTA tétrasodique | | 0,10 g |
| Carbopol 934 | | 0,30 g |
| triéthanolamine | | 0,30 g |
| butylhydroxytoluène | | 0,01 g |
| conservateur dans butylèneglycol : | | 5,00 g |
| phénoxyéthanol | 0,5 g | |
| 4-hydroxybenzoate de méthyle | 0,1 g | |
| 4-hydroxybenzoate d'éthyle | 0,1 g | |
| 4-hydroxybenzoate de propyle | 0,1 g | |
| 4-hydroxybenzoate de butyle | 0,1 g | |
| butylèneglycol | 4,1 g | |
| parfum | | 0,30 g |
| eau déminéralisée q.s.p. | | 100,00 g |

EXEMPLE 3

MICROEMULSION

| | |
|---|---|
| bétaïne | 0,10 g |
| chlorure de magnésium | 0,05 g |
| pyroglutamate de potassium | 0,10 g |
| REVITALINE | 0,50 g |
| Tween 60 | 20,00 g |
| glycérol | 28,00 g |
| dipélargonate de propylèneglycol | 40,00 g |
| 4-hydroxybenzoate d'éthyle | 0,30 g |
| parfum | 0,30 g |
| eau déminéralisée q.s.p. | 100,00 g |

La microémulsion est préparée en mélangeant l'ensemble des excipients et en les secouant jusqu'à l'obtention d'une solution limpide.

EXEMPLE 4

MICROEMULSION

| | |
|---|---|
| bétaïne | 0,20 g |
| gluconate de magnésium | 0,50 g |

| | | |
|---|---|---|
| gluconate de potassium | | 0,50 g |
| OXYDERMINE | | 0,50 g |
| Solutol HS 15 | | 1,00 g |
| labrafac hydrophile | | 0,25 g |
| Cétiol HE | | 0,20 g |
| Abil 8851 B | | 0,05 g |
| propylèneglycol | | 12,50 g |
| éthanol | | 12,50 g |
| polymère carboxypolyvinylique | | 0,40 g |
| parfum | | 0,30 g |
| conservateurs | | 0,30 g |
| colorant | q.s. | |
| eau déminéralisée | q.s.p. | 100,00 g |
| triéthanolamine | q.s.p. pH = 6 | |

La microémulsion est préparée comme décrit dans l'exemple 3.

EXEMPLE 5

BASE FLUIDE DE MAQUILLAGE

| | |
|---|---|
| bétaïne | 0,10 g |
| gluconate de magnésium | 0,90 g |
| pyroglutamate de potassium | 0,60 g |
| REVITALINE | 0,50 g |
| stérols de soja éthoxylés | 4,00 g |
| stérols de soja | 0,50 g |
| monostéarate de glycérol | 1,00 g |
| huile végétale | 1,50 g |
| palmitate d'éthyle-2 hexyle | 4,00 g |
| alcool cétylique | 0,50 g |
| triglycérides capriques/capryliques | 1,50 g |
| huile de silicone | 1,00 g |
| huile minérale | 1,80 g |
| alcools de lanoline | 0,20 g |
| dipélargonate de propylèneglycol | 3,00 g |
| lécithine | 1,00 g |

| | | |
|---|---|---|
| conservateur dans butylèneglycol : | | 5,00 g |
| phénoxyéthanol | 0,5 g | |
| 4-hydroxybenzoate de méthyle | 0,1 g | |
| 4-hydroxybenzoate d'éthyle | 0,1 g | |
| 4-hydroxybenzoate de propyle | 0,1 g | |
| 4-hydroxybenzoate de butyle | 0,1 g | |
| butylèneglycol | 4,1 g | |
| Carbopol 940 | | 0,20 g |
| triéthanolamine | | 0,20 g |
| EDTA tétrasodique | | 0,10 g |
| butylhydroxytoluène | | 0,01 g |
| parfum | | 0,30 g |

EXEMPLE 6

CREME DE NUIT

| | | |
|---|---|---|
| bétaïne | | 0,05 g |
| gluconate de magnésium | | 0,90 g |
| pyroglutamate de potassium | | 0,60 g |
| REVITALINE | | 0,50 g |
| alcool cétylique | | 2,00 g |
| stéarine | | 2,50 g |
| monostéarate de glycérol | | 5,00 g |
| palmitate d'isopropyle | | 5,00 g |
| huile végétale | | 3,00 g |
| huile minérale | | 2,00 g |
| perhydrosqualène | | 2,00 g |
| huile de silicone | | 1,00 g |
| beurre de karité | | 2,00 g |
| conservateur dans butylèneglycol : | | 5,00 g |
| phénoxyéthanol | 0,5 g | |
| 4-hydroxybenzoate de méthyle | 0,1 g | |
| 4-hydroxybenzoate d'éthyle | 0,1 g | |
| 4-hydroxybenzoate de propyle | 0,1 g | |
| 4-hydroxybenzoate de butyle | 0,1 g | |
| butylèneglycol | 4,1 g | |

| | | | |
|---|---|---|---|
| triéthanolamine | | | 5,50 g |
| EDTA tétrasodique | | | 0,10 g |
| parfum | | | 0,30 g |
| eau | q.s.p. | | 100,00 g |

EXEMPLE 7

GEL

| | | |
|---|---|---|
| bétaïne | | 0,10 g |
| gluconate de magnésium | | 0,90 g |
| gluconate de potassium | | 0,60 g |
| REVITALINE | | 0,50 g |
| Carbopol 940 | | 0,20 g |
| polyéthylèneglycol | | 3,00 g |
| conservateur dans butylèneglycol : | | 5,00 g |
| phénoxyéthanol | 0,5 g | |
| 4-hydroxybenzoate de méthyle | 0,1 g | |
| 4-hydroxybenzoate d'éthyle | 0,1 g | |
| 4-hydroxybenzoate de propyle | 0,1 g | |
| 4-hydroxybenzoate de butyle | 0,1 g | |
| butylèneglycol | 4,1 g | |
| Tween 20 | | 0,50 g |
| triéthanolamine | | 0,25 g |
| parfum | | 0,20 g |
| eau déminéralisée q.s.p. | | 100,00 g |

EXEMPLE 8

**SERUM**

| | |
|---|---|
| bétaïne | 0,10 g |
| gluconate de magnésium | 0,90 g |
| gluconate de potassium | 0,60 g |
| extrait de levure (VITACELL LS 1917) | 0,50 g |
| hydroxypropylméthylcellulose | 0,30 g |
| propylèneglycol | 5,00 g |
| glycérine | 5,00 g |
| alcool oléique éthoxylé | 0,50 g |
| parfum | 0,30 g |
| EDTA tétrasodique | 0,10 g |
| conservateurs | 0,50 g |
| albumine bovine | 5,00 g |
| eau déminéralisée q.s.p. | 100,00 g |

EXEMPLE 9

FOND DE TEINT

| | |
|---|---|
| bétaïne | 0,10 g |
| gluconate de magnésium | 0,90 g |
| pyroglutamate de potassium | 0,60 g |
| extrait de levure (VITACELL LS 1917) | 0,50 g |
| EDTA tétrasodique | 0,10 g |
| carboxyméthylcellulose | 0,20 g |
| silicate d'aluminium magnésium | 1,00 g |
| laurate de sorbitan éthoxylé | 1,00 g |
| propylèneglycol | 5,00 g |
| oxyde de titane | 2,00 g |
| talc | 1,00 g |
| pigments | 1,00 g |
| triéthanolamine | 0,50 g |
| conservateurs | 0,50 g |
| alcool cétylique | 1,00 g |
| alcool de lanoline | 3,00 g |
| acide stéarique | 1,80 g |
| monostéarate de propylèneglycol | 3,00 g |
| palmitate d'isopropyle | 8,00 g |
| huile végétale | 2,00 g |
| antioxydant | 0,05 g |
| parfum | 0,30 g |
| eau déminéralisée q.s.p. | 0,10 g |

**Revendications**

**1.** Composition cosmétique, caractérisée en ce qu'elle renferme un mélange :
(a) de 0,001 à 1 % de bétaïne,
(b) de 0,045 à 4,5 % d'ATP ou d'un système générateur d'ATP,
(c) de 10 ppb à 0,3 % de $Mg^{++}$,
(d) de 0,0001 à 0,6 % de $K^{+}$,
dans un excipient cosmétique, lesdits pourcentages étant exprimés en poids.

**2.** Composition cosmétique selon la revendication 1, caractérisée en ce que ledit mélange contient :
(a) de 0,03 à 0,3 % de bétaïne,
(b) de 0,15 à 1,5 % d'ATP ou d'un système générateur d'ATP,
(c) de 15 ppb à 0,1 % de $Mg^{++}$,
(d) de 0,001 à 0,2 % de $K^{+}$,
dans un excipient cosmétique, lesdits pourcentages étant exprimés en poids.

3. Composition cosmétique selon la revendication 2, caractérisée en ce que ledit mélange contient :
   (a) de 0,05 à 0,25 % de bétaïne,
   (b) de 0,25 à 1 % d'un système générateur d'ATP,
   (c) de 20 ppb à 0,06 % de $Mg^{++}$,
   (d) de 0,002 à 0,1 % de $K^{+}$,
   dans un excipient cosmétique, lesdits pourcentages étant exprimés en poids.

4. Composition cosmétique selon l'une quelconque des revendications 1 à 3, caractérisée en ce que le système générateur d'ATP est un extrait de cellules eucaryotes, en particulier de levures ou un extrait de rate bovine.

5. Composition selon l'une quelconque des revendications 1 à 4, caractérisée en ce que les sels de magnésium et de potassium sont choisis parmi les chlorures, les gluconates ou les pyroglutamates.

6. Utilisation d'une composition selon l'une quelconque des revendications 1 à 5 pour la préparation de compositions cosmétiques destinées à ralentir le vieillissement de la peau.

7. Procédé pour la préparation de compositions cosmétiques, destinées à ralentir le vieillissement de la peau, caractérisé en ce qu'il consiste à mélanger :
   (a) de la bétaïne,
   (b) de l'ATP ou un système générateur d'ATP,
   (c) un sel de magnésium et,
   (d) un sel de potassium,
   dans un excipient cosmétique.

8. Procédé selon la revendication 7, caractérisé en ce qu'il est mis en oeuvre pour la préparation de compositions cosmétiques selon l'une quelconque des revendications 1 à 5.

## Patentansprüche

1. Kosmetische Zusammensetzung, dadurch gekennzeichnet, daß sie ein Gemisch von
   a) 0,001 bis 1 % Betain,
   b) 0,045 bis 4,5 % ATP oder eines ATP-Erzeugungssystems,
   c) 10 ppb bis 0,3 % $Mg^{++}$,
   d) 0,0001 bis 0,6 % $K^{+}$
   in einem kosmetischen Excipiens enthält, wobei die Prozentangaben gewichtsbezogen sind.

2. Kosmetische Zusammensetzung nach Anspruch 1, dadurch gekennzeichnet, daß das Gemisch
   a) 0,03 bis 0,3 % Betain,
   b) 0,15 bis 1,5 % ATP oder eines ATP-Erzeugungssystems,
   c) 15 ppb bis 0,1 % $Mg^{++}$,
   d) 0,001 bis 0,2 % $K^{+}$
   in einem kosmetischen Excipiens enthält, wobei die Prozentangaben gewichtsbezogen sind.

3. Kosmetische Zusammensetzung nach Anspruch 2, dadurch gekennzeichnet, daß das Gemisch
   a) 0,05 bis 0,25 % Betain,
   b) 0,25 bis 1 % eines ATP-Erzeugersystems,
   c) 20 ppb bis 0,06 % $Mg^{++}$,
   d) 0,002 bis 0,1 % $K^{+}$
   in einem kosmetischen Excipiens enthält, wobei die Prozentangaben gewichtsbezogen sind.

4. Kosmetische Zusammensetzung nach einem der Ansprüche 1 bis 3, dadurch gekennzeichnet, daß das ATP-Erzeugungssystem ein Extrakt aus Eukaryotenzellen, insbesondere von Hefen, oder ein Extrakt von Rindermilz ist.

5. Zusammensetzung nach einem der Ansprüche 1 bis 4, dadurch gekennzeichnet, daß die Magnesium- und Kaliumsalze unter den Chloriden, Gluconaten und Pyroglutamaten ausgewählt sind.

6. Verwendung einer Zusammensetzung nach einem der Ansprüche 1 bis 5 zur Herstellung von kosmetischen Zusammensetzungen, die dafür bestimmt sind, das Altern der Haut zu verlangsamen.

7. Verfahren zur Herstellung kosmetischer Zusammensetzungen, die dafür bestimmt sind, das Altern der Haut zu verlangsamen, dadurch gekennzeichnet, daß es darin besteht,
   a) Betain,
   b) ATP oder ein ATP-Erzeugungssystem,
   c) ein Magnesiumsalz und
   d) ein Kaliumsalz

   in ein kosmetisches Excipiens einzumischen.

8. Verfahren nach Anspruch 7, dadurch gekennzeichnet, daß es zur Herstellung kosmetischer Zusammensetzungen nach einem der Ansprüche 1 bis 5 durchgeführt wird.

## Claims

1. Cosmetic composition characterized in that it comprises a mixture :
   (a) from 0.001 to 1 % of betaine,
   (b) from 0.045 to 4.5 % of ATP or an ATP generating system,
   (c) from 10 ppb to 0.3 % of $Mg^{++}$,
   (d) from 0.0001 to 0.6 % of $K^{+}$,

   in a cosmetic excipient, said percentages being by weight.

2. Cosmetic composition according to claim 1, characterized in that said mixture comprises :
   (a) from 0.03 to 0.3 % of betaine
   (b) from 0.15 to 1.5 % of ATP or an ATP generating system,
   (c) from 15 ppb to 0.1 % of $Mg^{++}$,
   (d) from 0.001 to 0.2 % of $K^{+}$,

   in a cosmetic excipient, said percentages being by weight.

3. Cosmetic composition according to claim 2, characterized in that said mixture comprises :
   (a) from 0.05 to 0.25 % of betaine,
   (b) from 0.25 to 1 % of an ATP generating system,
   (c) from 20 ppb to 0.06 % of $Mg^{++}$,
   (d) from 0.002 to 0.1 % of $K^{+}$,

   in a cosmetic excipient, said percentages being by weight.

4. Cosmetic composition according to any one of claims 1 to 3, characterized in that the ATP generating system, is an extract derived from eucaryotic cells, in particular from yeast or a bovine spleen extract.

5. Composition according to any one of claims 1 to 4, characterized in that the magnesium and potassium salts are selected from the chlorides, gluconates or pyroglutamates.

6. Use of a composition according to any one of claims 1 to 5 for preparing cosmetic compositions used for slowing ageing of the skin.

7. Method for preparing cosmetic compositions used for slowing ageing of the skin, characterized in that it consists in mixing :
   (a) betaine,
   (b) ATP or an ATP generating system,
   (c) a magnesium salt, and
   (d) a potassium salt,

   in a cosmetic excipient.

8. Method according to claim 7, characterized in that it is used in preparation of cosmetic compositions according to any one of claims 1 to 5.